# EUROPEAN PATENT SPECIFICATION

(11) **EP 1 248 623 B1**
(45) Date of publication and mention of the grant of the patent: **29.12.2004**
(21) Application number: 01900568.5
(22) Date of filing: 11.01.2001
(51) Int. Cl.: A61K 31/505, A61P 17/02

(54) **TREATMENT OF DIABETIC ULCERS**
BEHANDLUNG DIABETISCHER ULCERA
TRAITEMENT DES ULCERES DIABETIQUES

(30) Priority: 11.01.2000 GB 0000561
(43) Date of publication of application: 16.10.2002
(73) Proprietor: Pfizer Limited, Sandwich, Kent CT13 9NJ (GB); PFIZER INC., New York, N.Y. 10017 (US)
(72) Inventor: WOOD, Ralph, E., Moundsville, WV 26041 (US); DAVIES, Michael, John, Sandwich, Kent CT13 9NJ (GB); SIEGEL, Richard, Lewis, New York, NY 10017 (US)
(74) Representative: Motion, Keith Robert
(86) International application number: PCT/IB2001/000018
(87) International publication number: WO 2001/051042

(56) References cited:
- WO-A-99/54333
- BOULTON ANDREW J.M.: "End-stage complications of diabetic neuropathy: Foot ulceration" CANADIAN JOURNAL OF NEUROLOGICAL SCIENCES, vol. 21, no. 4, 1994, pages s18-s22, XP002164862

## Description

This invention relates to the use of cyclic guanosine 3', 5'-monophosphate type five (cGMP PDE5) inhibitors, including in particular the compound sildenafil, for the treatment of diabetic ulcers including in particular the treatment of chronic dermal ulcers such as diabetic foot ulcers.

Diabetes is a serious chronic disease. The 1996 global diabetes prevalence of 120 million is predicted to more than double to 250 million by the year 2025 due to increasing age, obesity, sedentary lifestyle and changing dietary patterns. Though many serious and costly complications. affect individuals with diabetes, such as heart disease, kidney failure and blindness, foot complications take the greatest toll: 40-70% of all lower extremity amputations are related to diabetes mellitus. Indeed, 85% of all diabetes-related lower extremity amputations are preceded by a foot ulcer.

Patients with diabetes mellitus are known to be at increased risk of developing chronic dermal ulcers such as an ulcer on the foot in the presence of established long-term complications of the disease. Ulceration occurs as the result of impaired nerve function (neuropathy) and/or in the presence of ischaemia.

Local tissue ischaemia is a major contributing factor to diabetic ulceration. As well as large vessel disease, patients with diabetes suffer additional threat to their skin perfusion by involvement of the non-conduit arteries in the process of atherosclerosis and, perhaps more importantly, impairment of the microcirculatory control mechanisms, so-called small vessel disease. Under normal circumstances, blood flow increases in response to injury to facilitate healing. In the presence of small vessel disease (and ischaemia) this response is significantly blunted and this, together with the tendency to thrombosis in the microcirculation during low flow, is probably important in ulcer pathogenesis.

Neuropathy is also a major complication of diabetes mellitus, with no well-established therapy for either its symptomatic treatment or for prevention of progressive decline in nerve function. Estimates of the prevalence of neuropathy in diabetes vary widely (5 to 80%), largely due to the wide variety of definitions and clinical descriptions of neuropathy. Nevertheless, prevalence rates in the order of 20% have been recorded in both hospital and community-based studies in the UK.

The effect of the neuropathy is complex. However, loss of sensory information from the foot undoubtedly contributes to abnormal and prolonged pressure on the areas of the foot (sensory neuropathy). Motor-neuropathy causes deformity, further increasing pressure loading. In autonomic neuropathy, loss of innovation of the sweat glands results in dry skin which cracks allowing foci for infection. Autonomic dysfunction contributes further by altering the distribution of microcirculatory blood flow, directing the blood flow through shunts and away from the nutritive skin capillaries. It is the interplay of these factors and foot trauma that result in the skin breakdown.

The mechanism leading to nerve damage in diabetes is not yet resolved but is almost certainly multifactorial, with genetic predisposition, metabolic and vascular abnormalities, and lack of perturbation of growth factors, implicated. The response of the peripheral nervous system to the metabolic insults received in diabetes does not seem to differ between type 1 and type 2 diabetes, suggesting the likelihood of similar clinical response to therapies in the two primary forms of the disease. There appears to be a number of susceptibility factors, as yet unknown, for the development of neuropathy, which operate in the presence of hyperglycaemia.

The invention also provides for the use of a cGMP PDE5 inhibitor for the manufacture of a composition for the treatment of diabetic ulcers.

The invention is of particular value for treating patients with diabetic foot ulceration.

A number of potent and selective cGMP PDE5 inhibitors are now known and their activity can be readily determined by in-vitro screening against cGMP PDE enzymes from a number of sources, in accordance with published procedures. Thus for example a number of pyrazolopyrimidinone compounds are described as cGMP PDE5 inhibitors in our European patents nos. 0463756 and 0526004; while further compounds are described in International patent applications nos. WO 93/12095; WO 94/05661; WO 94/00453; WO 95/19978 and WO 98/49166.

Preferred cGMP-PDE5 inhibitors for use in the present invention include:
5-(2-ethoxy-5-morpholinoacetylphenyl)-1-methyl-3-n-propyl-1-,6-dihydro-7H-pyrazolo[4, 3-d]pyrimidin-7-one;
5-(5-morpholinoacetyt-2-n-propoxyphenyt)-1-methyl-3-n-propyl-1-,6-dihydro-7H-pyrazolo[4, 3-d]pyrimidin-7-one;
5-[2-ethoxy-5-(4-methyl-1-piperazin-1-yl-sulphonyl)-phenyl]-1,6-dihydro-1-methyl-3-propylpyrazolo[4, 3-d]pyrimidin-7-one;
5-[2-allyloxy-5-(4-methyl-1-piperazinylsulphonyl)-phenyl]-1-methyl-3-n-propyl-1,6-dihydro-7H-pyrazolo[4, 3-d]pyrimidin-7-one;
5-(2-ethoxy-5-[4-(2-propyl)-1-piperazinyl-sulphonyl]phenyl}-1-methyl-3-n-propyl-1, 6-dihydro-7H-pyrazolo[4, 3-d)pyrimidin-7-one;
5-(2-ethaxy-5[4-(2-hydroxyethyl-1-piperazinylsulphonyl]phenyl)-1-methyl-3-n-propyl-1, 6-dihydro-7H-pyrazolo [4, 3-d]pyrimidin-7-one;
5-(5-[4-(2-hydroxyethyl)-1-piperazinylsulphonyl]-2-n-propoxyphenyl)-1-methyl-3-n-propyl-1, 6-dihydro-7H-pyrazolo[4, 3-d]pyrimidin-7-one;
5-[2-ethoxy-5-(4-methyl-1-piperazinylcarbonyl)-phenyl]-1-methyl-3-n-propyl-1,6-dihydro-7H-pyrazolo[4, 3-d]pyrimidin-7-one;
5-[2-ethoxy-5-(1-methyl-2-imidazolyl)phenyl]-1-methyf-3-n-propyl-1, 6-dihydro-7H-pyrzolo[4, 3-d]pyrimidin-7-one,
3-ethyl-5-[5-[4-ethylpiperazin-1-yl)sulphonyl]-2-(2-methoxyethoxy)pyrid-3-yl]-2-(2-pyridylmethyl)-6,7-dihydro-2H-pyrazolo[4,3-d]pyrimidin-7-one,
3-ethyl-5-[5-(4-ethylpiperazin-1-ylsulphonyl)-2-n-propoxyphenyl]-2-(pyridin-2-yl)methyl-2,6-dihydro-7H-pyrimidin-7-one, and
5-[2-ethoxy-5-(4-ethylpiperazin-1-ylsulphonyl)pyridin-3-yl]-3-ethyl-2-[2-methoxyethyl]-2,6-dihydro-7H-pyrazolo[4,3-d]pyrimidin-7-one, also known as 1-{6-ethoxy-5-[3-ethyl-6,7-dihydro-2-(2-methoxyethyl)-7-oxo-2H-pyrazolo[4,3-d]pyrimidin-5-yl]-3-pyridylsulphonyl}-4-ethylpiperazine (see Examples hereinafter for preparation).

Most particular preferred is the compound 5-[2-ethoxy-5-(4-methylpiperazin-1-yl sulphonyl)-phenyl]-1, 6-dihydro-1-methyl-3-propylpyrazolo [4,3-d] pyrimidine-7-one (sildenafil), and pharmaceutically acceptable salts thereof; including the citrate salt.

The suitability of any particular cGMP PDE5 inhibitor can be readily determined by evaluation of its potency and selectivity using literature methods followed by evaluation of its toxicity, absorption, metabolism, pharmacokinetics, etc in accordance with standard pharmaceutical practice.

Preferably, the cGMP PDE5 inhibitors have an IC50 for PDE5 at less than 100 nanomolar, more preferably, at less than 50 nanomolar, more preferably still at less than 10 nanomolar.

IC50 values for the cGMP PDE5 inhibitors may be determined using established literature methodology, for example as described in EP0463756-B1 and EP0526004-A1.

Preferably the cGMP PDE5 inhibitors used in the invention are selective for the PDE5 enzyme. Preferably they are selective over PDE3, more preferably over PDE3 and PDE4. Preferably, the cGMP PDE5 inhibitors of the invention have a selectivity ratio greater than 100 more preferably greater than 300, over PDE3 and more preferably over PDE3 and PDE4.

Selectivity ratios may readily be determined by the skilled person. IC50 values for the PDE3 and PDE4 enzyme may be determined using established literature methodology, see S A Ballard et al, Journal of Urology, 1998, vol. 159, pages 2164-2171.

The cGMP PDE5 inhibitors can be administered alone but, in human therapy will generally be administered in admixture with a suitable pharmaceutical excipient diluent or carrier selected with regard to the intended route of administration and standard pharmaceutical practice.

For example, the cGMP PDE5 inhibitors can be administered orally, buccally or sublingually in the form of tablets, capsules, ovules, elixirs, solutions or suspensions, which may contain flavouring or colouring agents, for immediate-, delayed-, modified-, or controlled-release applications.

Surprisingly, PDE5 inhibitors treat diabetic ulcers systemically. Accordingly, oral administration is a preferred route.

Such tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycollate, croscarmellose sodium and certain complex silicates, and granulation binders such as polyvinylpyrrolidone, hydroxypropylmethyl cellulose, hydroxypropylcellulose, sucrose, gelatin and acacia. Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the cGMP PDE5 inhibitors of the invention may be combined with various sweetening or flavouring agents, colouring matter or dyes, with emulsifying and/or suspending agents and with diluents such as water, ethanol, propylene glycol and glycerin, and combinations thereof.

The cGMP PDE5 inhibitors can also be administered parenterally, for example, intravenously, intra-arterially, intraperitoneally, intramuscularly or subcutaneously, or they may be administered by infusion techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

The dosage of cGMP PDE5 inhibitor in such formulations will depend on its potency, but can be expected to be in the range of from 1 to 500 mg for administration up to three times a day. For oral and parenteral administration to human patients, the daily dosage level of the cGMP PDE5 inhibitor will usually be from 5 to 500 mg (in single or divided doses). In the case of sildenafil, a preferred dose is in the range 10 to 100 mg (e.g. 10, 25, 50 and 100 mg) which can be administered once, twice or three times a day (preferably once). However the precise dose will be as determined by the prescribing physician and will depend on the age and weight of the patient and severity of the symptoms.

Thus, for example, tablets or capsules of the cGMP PDE5 inhibitor may contain from 5 to 250 mg (e.g. 10 to 100 mg) of active compound for administration singly or two or more at a time, as appropriate. The physician in any event will determine the actual dosage which will be most suitable for any individual patient and it will vary with the age, weight and response of the particular patient. The above dosages are exemplary of the average case. There can, of course, be individual instances where higher or lower dosage ranges are merited and such are within the scope of this invention.

The cGMP PDE5 inhibitors can also be administered intranasally or by inhalation and are conveniently delivered in the form of a dry powder inhaler or an aerosol spray presentation from a pressurised container, pump, spray or nebuliser with the use of a suitable propellant, e.g. dichlorodifluoromethane, trichlorofluoromethane, dichlorotetrafluoroethane, a hydrofluoroalkane such as 1,1,1,2-tetrafluoroethane or 1,1,1,2,3,3,3-heptafluoropropane, carbon dioxide or other suitable gas. In the case of a pressurised aerosol, the dosage unit may be determined by providing a valve to deliver a metered amount. The pressurised container, pump, spray or nebuliser may contain a solution or suspension of the cGMP PDE5 inhibitor, e.g. using a mixture of ethanol and the propellant as the solvent, which may additionally contain a lubricant, e.g. sorbitan trioleate. Capsules and cartridges (made, for example, from gelatin) for use in an inhaler or insufflator may be formulated to contain a powder mix of the cGMP PDE5 inhibitor and a suitable powder base such as lactose or starch.

Aerosol or dry powder formulations are preferably arranged so that each metered dose or "puff" contains from 1 to 50 mg of the cGMP PDE5 inhibitor, for delivery to the patient. The overall daily dose with an aerosol will be in the range of from 1 to 50 mg which may be administered in a single dose or, more usually, in divided doses throughout the day.

Alternatively, the cGMP PDE5 inhibitors can be administered in the form of a suppository or pessary.

The cGMP PDE5 inhibitor may be applied topically in the form of a gel, hydrogel, lotion, solution, cream, ointment or dusting powder. The cGMP PDE5 inhibitors may also be dermally or transdermally administered, for example, by the use of a skin patch.

Since diabetic ulcers occur on the skin surface, topical administration is a preferred route of administration.

For application topically to the skin, the cGMP PDE5 inhibitors can be formulated as a suitable ointment containing the inhibitor suspended or dissolved in, for example, a mixture with one or more of the following: mineral oil, liquid petrolatum, white petrolatum, propylene glycol, polyoxyethylene polyoxypropylene compound, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The cGMP PDE5 inhibitors may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as,an auxiliary additive, e.g. as a carrier, diluent or solubiliser. Alpha-, beta- and gamma-cyclodextrins are most commonly used and suitable examples are described in WO-A-91/11172, WO-A-94/02518 and WO-A-98/55148.

Generally, in humans, oral administration of the cGMP PDE5 inhibitors is the preferred route, being the most convenient. In circumstances where the recipient suffers from a swallowing disorder or from impairment of drug absorption after oral administration, the drug may be administered parenterally, sublingually or buccally.

It is to be appreciated that all references herein to treatment include curative, palliative and prophylactic treatment.

The following formulation examples are illustrative only and are not intended to limit the scope of the invention . Active ingredient means a cGMP PDE5 inhibitor.

### Formulation 1:

A tablet is prepared using the following ingredients :
Sildenafil citrate (50 mg) is blended with cellulose (microcrystalline), silicon dioxide, stearic acid (fumed) and the mixture is compressed to form tablets.

### Formulation 2 :

An intravenous formulation may be prepared by combining active ingredient (100 mg) with isotonic saline (1000 ml).

The efficacy of the cGMP PDE5 inhibitors in treating neuropathy in human patients is illustrated by the following anecdotal report.

The patient was an insulin-dependent diabetic who had been suffering from erectile dysfunction and who subsequently developed a diabetic foot ulcer. During treatment of his erectile dysfunction with sildenafil it was noted that his foot ulcer, which he had had for approximately two years, was healing. The patient had been through various courses of treatment with minimal results. He had seen vascular surgeons, podiatrists and had visited wound care clinics. Ultimately, he was hopitalised for approximately one month on IV antibiotics etc, and the threat was very real that the patient was going to require a below-the-knee amputation. At times the ulcer would appear to be healing only to re-occur, returning to its pre-treatment size and depth. Once Sildenafil treatment had begun for his erectile dysfunction, it was noted that the ulcer was decreasing in size. The patient was instructed to continue taking 50 mg of Sildenafil once a day. This treatment resulted in complete resolution of the diabetic foot ulcer within one month. The patient has continued on this same treatment for the past two years without re-occurrence.

### Examples

### 2-(Methoxyethyl)-5-[2-ethoxy-5-(4-ethylpiperazin-1-ylsulphonyl)pyridin-3-yl]-3-ethyl-2,6-dihydro-7H-pyrazolo[4,3-d]pyrimidin-7-one

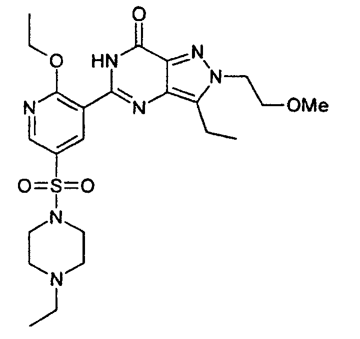

A mixture of the product from stage i) below (0.75mmol), potassium bis(trimethylsilyl)amide (298mg, 1.50mmol) and ethyl acetate (73 microlitres, 0.75mmol) in ethanol (10ml) was heated at 120°C in a sealed vessel for 12 hours. The cooled mixture was partitioned between ethyl acetate and aqueous sodium bicarbonate solution, and the layers separated. The organic phase was dried (MgSO₄), and evaporated under reduced pressure. The crude product was purified by column chromatography on silica gel using dichloromethane:methanol (98:2) as eluant to afford the title compound, 164mg; Found : C, 53.18; H, 6.48; N, 18.14; C₂₃H₃₃N₇O₅S;0.20C₂H₅CO₂CH₃ requires C, 53.21; H, 6.49; N, 18.25%; δ (CDCl₃) : 1.04 (3H, t), 1.40 (3H, t), 1.58 (3H, t), 2.41 (2H, q), 2.57 (4H, m), 3.08 (2H, q), 3.14 (4H, m), 3.30 (3H, s), 3.92 (2H, t), 4.46 (2H, t), 4.75 (2H, q), 8.62 (1H, d), 9.04 (1H, d), 10.61 (1H, s); LRMS : m/z 520 (M+1)⁺ ; mp 161-162°C.

### Preparation of Starting Materials

### a) Pyridine-2-amino-5-sulphonic acid

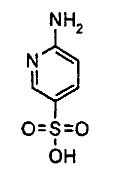

2-Aminopyridine (80g, 0.85mol) was added portionwise over 30 minutes to oleum (320g) and the resulting solution heated at 140°C for 4 hours. On cooling, the reaction was poured onto ice (200g) and the mixture stirred in an ice/salt bath for a further 2 hours. The resulting suspension was filtered, the solid washed with ice water (200ml) and cold IMS (200ml) and dried under suction to afford the title compound as a solid, 111.3g; LRMS : m/z 175 (M+1)⁺.

### b) Pyridine-2-amino-3-bromo-5-sulphonic acid

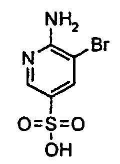

Bromine (99g, 0.62mol) was added dropwise over an hour, to a hot solution of the product from stage a) (108g, 0.62mol) in water (600ml) so as to maintain a steady reflux. Once the addition was complete the reaction was cooled and the resulting mixture filtered. The solid was washed with water and dried under suction to afford the title compound, 53.4g; δ (DMSOd_{6,} 300MHz): 8.08 (1H, s), 8.14 (1H, s); LRMS : m/z 253 (M)⁺.

### c) Pyridine-3-bromo-2-chloro-5-sulphonyl chloride

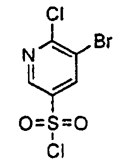

A solution of sodium nitrite (7.6g, 110.0mmol) in water (30ml) was added dropwise to an ice-cooled solution of the product from stage b) (25.3g, 100.0mmol) in aqueous hydrochloric acid (115ml, 20%), so as to maintain the temperature below 6°C. The reaction was stirred for 30 minutes at 0°C and for a further hour at room temperature. The reaction mixture was evaporated under reduced pressure and the residue dried under vacuum at 70°C for 72 hours. A mixture of this solid, phosphorus pentachloride (30.0g, 144mmol) and phosphorus oxychloride (1ml, 10.8mmol) was heated at 125°C for 3 hours, and then cooled. The reaction mixture was poured onto ice (100g) and the resulting solid filtered, and washed with water. The product was dissolved in dichloromethane, dried (MgSO₄), and evaporated under reduced pressure to afford the title compound as a yellow solid, 26.58g; δ (CDCl_{3,} 300MHz) : 8.46 (1H, s), 8.92 (1H, s).

### d) 3-Bromo-2-chloro-5-(4-ethypiperazin-1-ylsulphonyl)pyridine

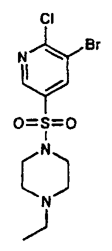

A solution of 1-ethylpiperazine (11.3ml, 89.0mmol) and triethylamine (12.5ml, 89.0mmol) in dichloromethane (150ml) was added dropwise to an ice-cooled solution of the product from stage c) (23.0g, 79.0mmol) in dichloromethane (150ml) and the reaction stirred at 0°C for an hour. The reaction mixture was concentrated under reduced pressure and the residual brown oil was purified by column chromatography on silica gel, using an elution gradient of dichloromethane:methanol (99:1 to 97:3) to afford the title compound as an orange solid, 14.5g; δ (CDCl₃, 300MHz) : 1.05 (3H, t), 2.42 (2H, q), 2.55 (4H, m), 3.12 (4H, m), 8.24 (1H, s), 8.67 (1H, s).

### e) 3-Bromo-2-ethoxy-5-(4-ethylpiperazin-1-ylsulphonyl)pyridine

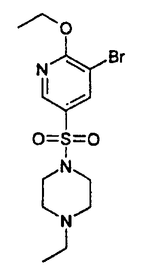

A mixture of the product from stage d) (6.60g, 17.9mmol) and sodium ethoxide (6.09g, 89.55mmol) in ethanol (100ml) was heated under reflux for 18 hours, then cooled. The reaction mixture was concentrated under reduced pressure, the residue partitioned between water (100ml) and ethyl acetate (100ml), and the layers separated. The aqueous phase was extracted with ethyl acetate (2x100ml), the combined organic solutions dried (MgSO₄) and evaporated under reduced pressure to afford the title compound as a brown solid, 6.41g; Found : C, 41.27; H, 5.33; N, 11.11. C₁₃H₂₀BrN₃O₃S requires C, 41.35; H, 5.28; N, 10.99%; δ (CDCl_{3.} 300MHz) : 1.06 (3H, t), 1.48 (3H, t), 2.42 (2H, q), 2.56 (4H, m), 3.09 (4H, m), 4.54 (2H, q), 8.10 (1H, s), 8.46 (1H, s); LRMS : m/z 378, 380 (M+1)⁺.

### f) Pyridine 2-ethoxy-5-(4-ethylpiperazin-1-ylsulphonyl)-3-carboxylic acid ethyl ester

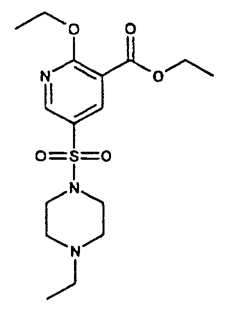

A mixture of the product from stage e) (6.40g, 16.92mmol), triethylamine (12ml, 86.1mmol), and palladium (0) tris(triphenylphosphine) in ethanol (60ml) was heated at 100°C and 200 psi, under a carbon monoxide atmosphere, for 18 hours, then cooled. The reaction mixture was evaporated under reduced pressure and the residue purified by column chromatography on silica gel, using an elution gradient of dichloromethane:methanol (100:0 to 97:3) to afford the title compound as an orange oil, 6.2g; δ (CDCl_{3,} 300MHz) : 1.02 (3H, t), 1.39 (3H, t), 1.45 (3H, t), 2.40 (2H, q), 2.54 (4H, m), 3.08 (4H, m), 4.38 (2H, q), 4.55 (2H, q), 8.37 (1H, s), 8.62 (1H, s); LRMS : m/z 372 (M+1)⁺

### g) Pyridine 2-ethoxy-5-(4-ethylpiperazin-1-ylsulphonyl)-3-carboxylic acid

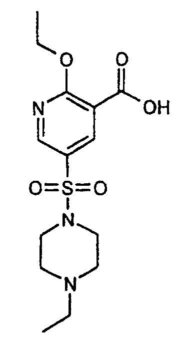

A mixture of the product from stage f) (4.96g, 13.35mmol) and aqueous sodium hydroxide solution (25ml, 2N, 50.0mmol) in ethanol (25ml) was stirred at room temperature for 2 hours. The reaction mixture was concentrated under reduced pressure to half it's volume, washed with ether and acidified to pH 5 using 4N hydrochloric acid. The aqueous solution was extracted with dichloromethane (3x30ml), the combined organic extracts dried (MgSO₄) and evaporated under reduced pressure to afford the title compound as a tan coloured solid, 4.02g; δ (DMSOd_{6,} 300MHz) : 1.18 (3H, t), 1.37 (3H, t), 3.08 (2H, q), 3.17-3.35 (8H, m), 4.52 (2H, q), 8.30 (1H, s), 8.70 (1H, s).

### h) 4-[2-Ethoxy-5-(4-ethylpiperazin-1-ylsulphonyl)pyridin-3-ylcarboxamido]-1H-3-ethylpyrazole-5-carboxamide

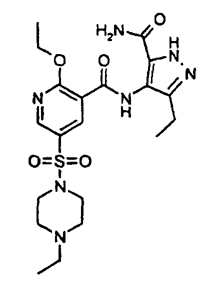

A solution of 4-amino-3-ethyl-1H-pyrazole-5-carboxamide (WO 9849166) (9.2g, 59.8mmol) in N,N-dimethylformamide (60ml) was added to a solution of the product from stage g) (21.7g, 62.9mmol), 1-hydroxybenzotriazole hydrate (10.1g, 66.0mmol) and triethylamine (13.15ml, 94.3mmol) in dichloromethane (240ml). 1-(3-Dimethylaminopropyl)-3-ethylcarbodiimide hydrochloride (13.26g, 69.2mmol) was added and the reaction stirred at room temperature for 6 hours. The dichloromethane was removed under reduced pressure, the remaining solution poured into ethyl acetate (400ml), and this mixture washed with aqueous sodium bicarbonate solution (400ml). The resulting crystalline precipitate was filtered, washed with ethyl acetate and dried under vacuum, to afford the title compound, as a white powder, 22g; δ (CDCl₃+1 drop DMSOd₆) 0.96 (3H, t), 1.18 (3H, t), 1.50 (3H, t), 2.25-2.56 (6H, m), 2.84 (2H, q), 3.00 (4H, m), 4.70 (2H, q), 5.60 (1H, br s), 6.78 (1H, br s), 8.56 (1H, d), 8.76 (1H, d), 10.59 (1H, s), 12.10-12.30 (1H, s); LRMS: m/z 480 (M+1)⁺.

### i) 2-Methoxyethyl-4-[2-ethoxy-5-(4-ethylpiperazin-1-ylsulphonyl)pyridin-3-ylcarboxamido]-3-ethylpyrazole-5-carboxamide

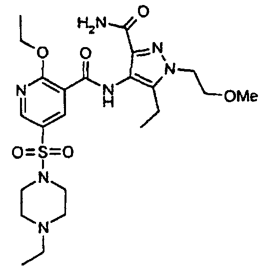

1-Bromo-2-methoxyethane (1.72mmol) was added to a solution of the product from stage h) (750mg, 1.56mmol) and caesium carbonate (1.12g, 3.44mmol) in N,N-dimethylformamide (15ml) and the reaction stirred at 60° C for 18 hours. The cooled mixture was partitioned between water and ethyl acetate, and the layers separated. The organic layer was dried (MgSO₄), concentrated under reduced pressure and azeotroped with toluene to give a solid. This product was recrystallised from ether, to afford the title compound as a white solid.

## Claims

1. The use of a cGMP PDE5 inhibitor for the manufacture of a pharmaceutical composition for the treatment of diabetic ulcers.

2. Use as claimed in claim 1 wherein the diabetic ulcers are foot ulcers.

3. Use as claimed in claim 1 or claim 2 wherein the cGMP PDE5 inhibitor is 5-[2-ethoxy-5-(4-methylpiperazin-1-yl sulphonyl)-;phenyl]-1,6-dihydro-1-methyl-3-propylpyrazolo[4,3-d]pyrimidin-7-one.

4. Use as claimed in claim 1 or claim 2 wherein the cGMP PDE5 inhibitor is 5-[2-ethoxy-5-(4-ethylpiperazin-1-yl sulphonyl)pyridin-3-yl]-3-ethyl-2-[2-methoxyethyl]-2,6-dihydro-7H-pyrazolo[4,3-d]pyrimidin-7-one.

## Patentansprüche

1. Verwendung eines cGMP PDE5-Inhibitors zur Herstellung einer pharmazeutischen Zusammensetzung für die Behandlung von diabetischen Geschwüren.

2. Verwendung nach Anspruch 1, wobei die diabetischen Geschwüre Fußgeschwüre sind.

3. Verwendung nach Anspruch 1 oder Anspruch 2, wobei der cGMP PDE5-Inhibitor 5-[2-Ethoxy-5-(4-methylpiperazin-1-yl-sulfonyl)-phenyl]-1,6-dihydro-1-methyl-3-propylpyrazolo[4,3-d]pyrimidin-7-on ist.

4. Verwendung nach Anspruch 1 oder Anspruch 2, wobei der cGMP PDE5-Inhibitor 5-[2-Ethoxy-5-(4-ethylpiperazin-1-yl-sulfonyl)pyridin-3-yl]-3-ethyl-2-[2-methoxyethyl]-2,6-dihydro-7H-pyrazolo[4,3-d]pyrimidin-7-on ist.

## Revendications

1. Utilisation d'un inhibiteur de cGMP PDE5 pour la production d'une composition pharmaceutique destinée au traitement des ulcères diabétiques.

2. Utilisation suivant la revendication 1, dans laquelle les ulcères diabétiques sont des ulcères des pieds.

3. Utilisation suivant la revendication 1 ou la revendication 2, dans laquelle l'inhibiteur de cGMP PDE5 est la 5-[2-éthoxy-5-(4-méthylpipérazine-1-yl-sulfonyl)-phényl]-1,6-dihydro-1-méthyl-3-propylpyrazolo[4,3-d]pyrimidine-7-one.

4. Utilisation suivant la revendication 1 ou la revendication 2, dans laquelle l'inhibiteur de cGMP PDE5 est la 5-[2-éthoxy-5-(4-éthylpipérazine-1-yl-sulfonyl)-pyridine-3-yl]-3-éthyl-2-[2-méthoxyéthyl]-2,6-dihydro-7H-pyrazolo[4,3-d]pyrimidine-7-one.
